# EUROPEAN PATENT APPLICATION

(11) **EP 2 810 675 A1**
(43) Date of publication of application: **10.12.2014**
(21) Application number: 13382212.2
(22) Date of filing: 04.06.2013
(51) Int. Cl.: A61M 5/24, A61F 9/00

(54) **Injector for intraocular injection**

(71) Applicant: Bioftalmik, S.L., 48160 Derio-Vizcaya (ES); Reiner Microtek S.L., 20820 Deba- Guipuzcoa (ES); Corcostegui Crespo, Inigo, 48006 Bilbao -Vizcaya (ES)
(72) Inventor: Ccorcóstegui Crespo, Iñigo, E-48006 Bilbao -Vizcaya (ES); Careaga Goicoechea, Jon, E-48160 Derio-Vizcaya (ES); Igarza, Javier, E-20820 Deba- Guipúzcoa (ES)
(74) Representative: ABG Patentes, S.L.

(57) **Abstract**

The present invention relates to an injector (1) for intraocular injection comprising a cylinder (3) comprising a needle connecting hole (6) at one end and a movable plunger (4) tightly fitted within the cylinder (3), characterized in that it comprises a casing element (5) arranged outside the cylinder (3), suitable for sliding over the outer surface of the cylinder (3) and attached to the plunger (4).

## Description

### Field of the Invention

The present invention is comprised in the field of medical devices for performing intraocular injections that are completely safe for the patient and comfortable for the doctor when performing the injection.

### Background

Today, a professional, for example an ophthalmologist, must perform conventional intraocular injections with both hands: one hand keeping the eye of the patient open and the other hand introducing needle into the eyeball. This forces him/her to release the hand which is holding the eye of the patient open at the time of injection (which involves a risk for the patient if he/she moves his/her eye) in order to push the plunger of the syringe, or to have an assistant which increases the risk of clinical handling.

In recent years, the tendency to administer drugs by intraocular injection has grown exponentially as a result of the introduction on the market of new dosage forms for treating age-associated macular degeneration with anti-VEGF. In turn, the number of vitrectomies performed is also on the rise with the subsequent need of injecting new, more advanced contrast agents which improve viewing the inside of the eyeball and its inner structures.

Among the new drugs that have been launched on the market and those that are in the marketing phase, there are some which incorporate pre-filled injection kits similar to the commercially available injection syringes for injection with both hands. Others require the use of commercially available syringes. In these cases, the small volumes to be injected into the eyeball require the use of low capacity intravenous injection syringes (syringes for diabetics) that were not specifically designed for intraocular injections.

The current intraocular injection method can be described according to the following steps:
1. The patient receives topical anesthesia, such that he/she is alert and can move his/her eye. His/her eyelid is secured with a metallic system, for example,
2. The professional positions the syringe with his right hand (for right-handed doctors, and vice versa for left-handed doctors) and introduces the needle while holding down the patient's head.
3. The professional removes his/her hand from the eye of the patient: the professional moves his/her left hand (for right-handed doctors) in front of his/her line of sight to put his/her hand on the plunger.
4. The professional pushes the plunger with the subsequent risk of losing the puncture angle and the correct view of product discharge. Recommendations indicate that the injection is applied at a predetermined distance between 3 and 4 millimeters from the tangent of the limbus (transition area between the cornea and the conjunctiva of the eye). This injection point is critical because it is necessary to prevent causing damage to the patient, which may even be irreversible. The depth must also be controlled in order to inject the drug in the suitable area.

The support of an assistant who presses on the plunger while the ophthalmologist keeps the eye of the patient still and the syringe at the required injection angle is often required to facilitate the method.

The method requires a series of considerations:
- injections must always be made in the suitable position that is comfortable for the doctor and painless for the patient
- the professional positions the injector with his/her right hand (for right-handed doctors, and vice versa for left-handed doctors) and introduces the syringe while at the same time holding the patient's head with the other hand, and then once in a position suitable for injection, he/she moves his/her left hand in front of his/her line of sight and pushes the plunger for injection. This has the risks of the left hand lacking sensitivity for injecting an object held with the right hand and of temporarily losing the line of sight,
- the professional or doctor must perform the entire procedure at a certain speed, keeping the eyeball as still as possible throughout the intervention,
- the puncture must be made at a specific distance of 3 to 4 millimeters from the corneal limbus and at a maximum depth to prevent structural damage inside the eyeball,
- the puncture must be made with a specific inclination of about 45° with respect to the conjunctival epithelium surface, maintaining this inclination throughout the entire procedure to prevent touching the intraocular structures,
- the puncture point, syringe inclination and the emptying of the drug to be incorporated into the eyeball must be in sight at all times.

In the state of the art, all these requirements depend on the skill of the professional, with the subsequent technical problem of putting the procedure under the full responsibility of the professional and the risk of human error.

Commercially available syringes that are most recommendable today for such interventions are syringes used in diabetes due to their volume of between 0.5 and 1 milliliter.

### Description of the Invention

The technical problems mentioned in the state of the art are overcome by means of an injector according to claim 1. The particular embodiments of the invention are defined in the dependent claims.

A first inventive aspect relates to an *injector for intraocular injection comprising*
*a cylinder comprising a needle connecting hole at one end*, *and*
*a movable plunger tightly fitted within the cylinder*,
***characterized in that** it comprises*
*a casing element arranged outside the cylinder, suitable for sliding over the outer surface of the cylinder and attached to the plunger.*

The injector according to the invention can be used for intraocularly injecting any liquid substance, suspension, reconstituted vial or drug with only one hand. In the case of placing a needle, this device allows both making a puncture using a needle placed within the cylinder, and moving the plunger to discharge an active ingredient into the eye with only one hand, thus preventing the doctor from removing his/her hand which is holding the eye of the patient open. That is possible as a result of a sliding casing element. This improves the practice and provides more safety to the act of injection and to the patient. The sliding occurs when the specialist or doctor pushes the plunger with his/her finger, for example.

The sliding casing element provides the technical advantage of moving the plunger since it is attached to same. This element can slide over the cylinder and move the plunger.

The plunger is tightly fitted within the cylinder such that it is capable of pushing any substance inside the cylinder.

All the features described in the present document (including the claims, description and drawings) can be combined in any combination except those that are mutually exclusive.

### Description of the Drawings

The foregoing and other features and advantages of the invention will be more clearly understood in view of the detailed description comprising preferred embodiments shown by way of a non-limiting example in reference to the drawings.

Figure 1 shows a perspective view of an embodiment of the injector (1) according to the invention where the cylinder (3), plunger (4), needle connecting hole (6), the holding element (2) with non-slip elements (9) and the casing element (5) with gripping elements (8) and auxiliary sliding elements (7) on both sides of the casing (5) are seen.

Figure 2 shows an elevational view of an embodiment of the injector (1) according to the invention.

Figure 3 shows an elevational view of an embodiment of the injector (1) according to the invention and rotated 90 degrees with respect to Figure 2.

Figure 4 shows an elevational view of an embodiment of the injector (1) according to the invention, rotated 180 degrees with respect to Figure 3 and where the holding element (2) and the auxiliary sliding elements (7) are seen.

Figure 5 shows a front elevational view of an embodiment of the injector (1), where the needle connecting hole (6) and the holding element (2) are seen.

Figure 6 shows a perspective view of an embodiment of part of an injector (1) according to the invention, where elements such as positioning element (10) and needle stop element (11) are shown.

### Detailed Description of the invention

After having described the object of the invention, specific non-limiting embodiments thereof are detailed below.

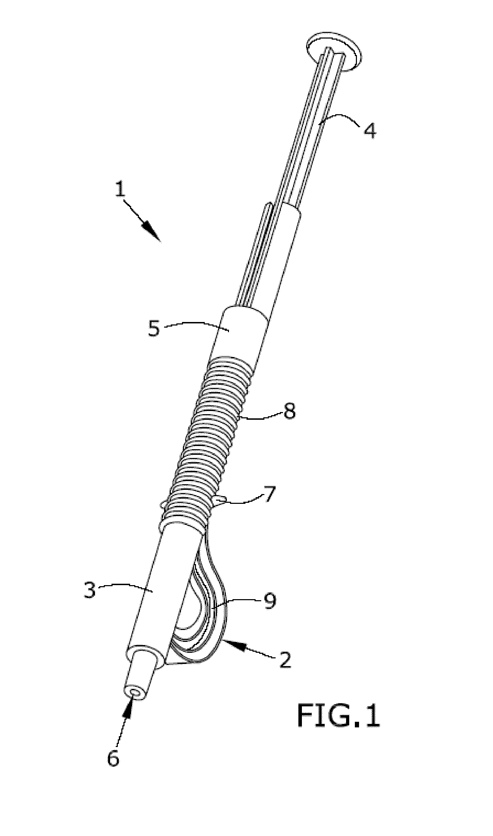

In a first embodiment shown in Figure 1, the injector (1) for intraocular injection comprises a cylinder (3) comprising a needle connecting hole (6) at one end and a movable plunger (4) tightly fitted within the cylinder (3). The injector (1) in turn comprises a casing element (5) arranged outside the cylinder (3), suitable for sliding over the outer surface of the cylinder (3) and attached to the plunger (4).

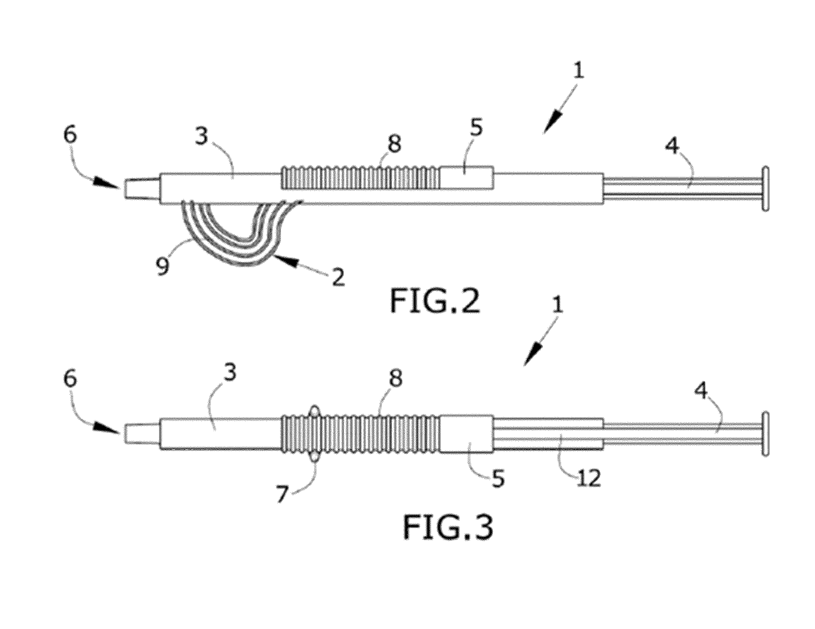

In the embodiment depicted in Figure 1, the casing element (5) has the shape of a hollow cylinder concentric to the cylinder (3). Advantageously, the casing element (5) is attached to the plunger (4) by means of an attachment section (not depicted) running on a channel (12) provided in the cylinder (3) such as in the manner shown in Figure 3, for example.

In one embodiment, the casing element (5) comprises at least one flange or projection-like auxiliary element (7) for the sliding for the sliding which allows resting a finger therein as pressure is exerted while emptying the injector (1) and sliding the plunger (4) with only one finger, for example. This auxiliary element (7) for the sliding aids in pushing the plunger (4) with only one hand. Particularly, the auxiliary element (7) for the sliding is located on one side of the cylinder (3). In one embodiment, the injector (1) comprises two auxiliary elements (7) located on both sides of the casing (5) thus allowing it to be used both with the right or left hand, for right-handed and left-handed users alike. Advantageously, in this last embodiment, both auxiliary sliding elements (7) are placed diametrically opposite one another.

On the other hand, in one embodiment the injector (1) comprises a holding element (2), for example, an ergonomic flange (2) as shown in Figure 1, which allows holding the injector (1) with the thumb and middle finger during injection, for example.

In one embodiment, the holding element (2) comprises non-slip elements (9). In one embodiment, the non-slip elements (9) are strata of different thickness as can be seen in Figure 2. The strata of different thickness prevent the movement of the fingers during the application of movement towards the needle connecting hole (6) which occurs when moving the plunger (4). In a particular example, the decreasing thickness of the strata of different thickness decreases from the side closest to the casing element (5).

As indicated, the injector (1) comprises a sliding casing (5) for moving the plunger (4) by means of pressure, for example, with the index finger, such that said pressure is exerted towards the needle connecting hole (6). Therefore in a particular embodiment, the casing element (5) comprises at least one gripping element (8) to prevent the finger from sliding along same. This gripping element (8) has the technical effect of aiding the gripping in order to push the plunger (4), for example, with the index finger. Advantageously, the casing element (5) comprises a plurality of non-slip projections (8) forming a non-slip zone.

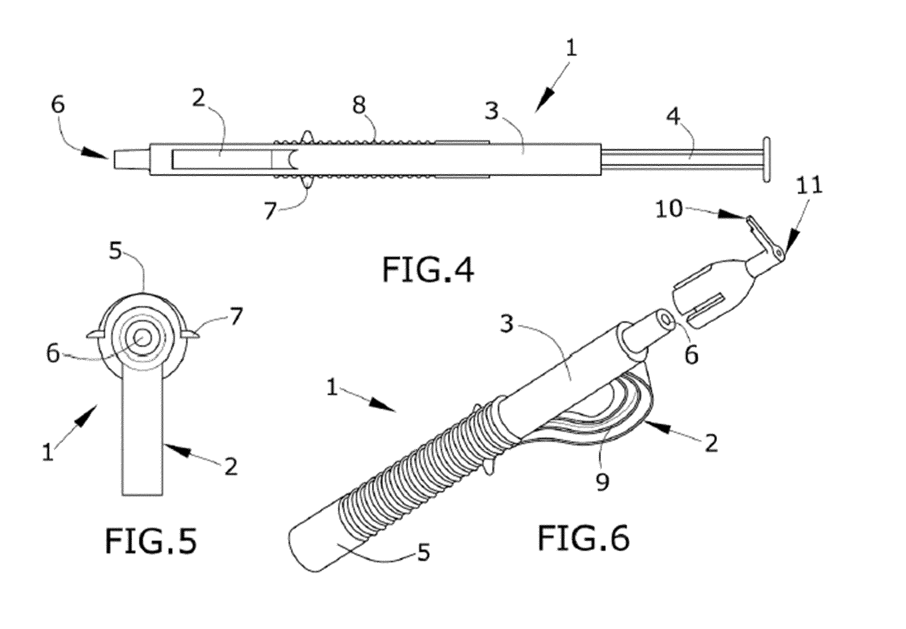

In one embodiment of the invention as shown in Figure 6, the injector (1) comprises a positioning element (10) coupleable to the needle to be used with the injector (1), comprising a needle stop element (11) to facilitate making a puncture at the exact required distance from the corneal limbus and with the pre-established depth to prevent intraocular structure damage due to aggression.

Other advantages of the intraocular injector (1) of the invention are:
- it allows having one hand free for keeping the eye of the patient still during the action of injecting the drug,
- it allows locating the puncture point at the exact distance from the corneal limbus and at a maximum depth to prevent damaging structures inside the eyeball,
- it facilitates the controlled maintenance of a 45° puncture angle throughout the entire intervention since it does not involve the interference of the other hand or of third parties during the process of moving the plunger (4),
- it allows viewing the puncture point, the inclination thereof and the emptying of the content to incorporated into the eyeball at all times,
- it allows performing any type of ocular injection, be it a subconjunctival injection, a palpebral injection, etc...
- it allows performing any type of injection involving the use of only one hand.

A method for performing an intraocular injection by means of an injector (1) according to the invention allows discharging the content in a controlled manner into an eye that is kept completely still with a suitable puncture positioning and depth, and a full view throughout the entire intervention without requiring the external aid of an assistant to perform the injection. All this significantly facilitates the surgical practice and prevents running unnecessary risks which may affect the inner structures of the eye or may cause unnecessary pain in the patient

In a particular embodiment of the invention, the method comprises the steps of:
a) providing an ocular injector according to the first inventive aspect comprising a drug or medicinal product,
b) holding the injector through a securing element (flange) located in the front part of the cylinder, preferably with the thumb and middle finger,
c) positioning a needle placed in the needle connecting hole (6) into the eye of a patient in a correct position and with a specific depth,
d) pressing, preferably with the index finger, on the casing element (5) such that the casing element (5) moves the plunger (4) to discharge the drug into the eye.

In a particular embodiment of the method, in step b) the injector is preferably held with the thumb and middle finger through the holding element (2). This allows exploiting the technical advantages of the holding element (2).

In a particular embodiment of the method, in step c) the position where the needle is placed is determined by the positioning element (10) which is adapted to the injector once the needle is coupled. This allows exploiting the technical advantages of the positioning element (10) as the needle is separated a specific distance from the corneal limbus.

In a particular embodiment of the method, in step c) the depth at which the needle is injected into the eye is determined by the needle stop element (11). This allows exploiting the technical advantages of the needle stop element (11).

In a particular embodiment of the method, in step d) pressure is exerted, preferably with the index finger, on the auxiliary element (7) for the sliding. This allows both left-handed and right-handed users alike to exploit the technical advantages of the auxiliary element (7) for the sliding.

## Claims

1. An injector (1) for intraocular injection comprising,
a cylinder (3) comprising a needle connecting hole (6) at one end, and
a movable plunger (4) tightly fitted within the cylinder (3),
**characterized in that** it comprises
a casing element (5) arranged outside the cylinder (3), suitable for sliding over the outer surface of the cylinder (3) and attached to the plunger (4).

2. The injector (1) according to claim 1, **characterized in that** it comprises a holding element (2).

3. The injector (1) according to any one of the preceding claims, **characterized in that** the casing element (5) comprises at least one auxiliary element (7) for the sliding.

4. The injector (1) according to any one of the preceding claims, **characterized in that** the casing element (5) comprises at least one gripping element (8).

5. The injector (1) according to claim 4, **characterized in that** holding element (2) comprises non-slip elements (9).

6. The injector (1) according to claim 5, **characterized in that** the non-slip elements (9) are strata of different thickness.

7. The injector (1) according to claim 1, **characterized in that** the casing element (5) is attached to the plunger (4) by means of an attachment section running on a channel (12) provided in the cylinder (3).

8. The injector (1) according to any one of the preceding claims, **characterized in that** it comprises a needle stop element (11).

9. The injector (1) according to any one of the preceding claims, **characterized in that** it comprises a positioning element (10).
